Europäisches Patentamt

(19) European Patent Office

Office européen des brevets

(11) Publication number: **0 015 680**
**B1**

(12) **EUROPEAN PATENT SPECIFICATION**

(45) Date of publication of patent specification: **30.06.82**

(51) Int. Cl.³: **C 12 M 1/04**

(21) Application number: **80300487.8**

(22) Date of filing: **20.02.80**

(54) Fermentation process and apparatus.

<table>
<tr><td>(30) Priority: <b>09.03.79 GB 7908358</b></td><td>(73) Proprietor: <b>IMPERIAL CHEMICAL INDUSTRIES PLC</b><br><b>Imperial Chemical House Millbank</b><br><b>London SW1P 3JF (GB)</b></td></tr>
<tr><td>(43) Date of publication of application:<br><b>17.09.80 Bulletin 80/19</b></td><td></td></tr>
<tr><td></td><td>(72) Inventor: <b>Shipley, David Graham</b><br><b>104 Darlington Road Hartburn</b><br><b>Stockton-on-Tees Cleveland (GB)</b></td></tr>
<tr><td>(45) Publication of the grant of the patent:<br><b>30.06.82 Bulletin 82/26</b></td><td></td></tr>
<tr><td>(84) Designated Contracting States:<br><b>BE DE FR GB IT NL SE</b></td><td>(74) Representative: <b>Aufflick, James Neil et al,</b><br><b>Imperial Chemical Industries PLC Legal Department: Patents Thames House North Millbank</b><br><b>London SW1P 4QG (GB)</b></td></tr>
<tr><td>(56) References cited:<br><b>AT - A - 149 228<br>DE - A - 1 936 350<br>DE - A - 2 438 551<br>FR - A - 1 529 494<br>FR - A - 2 118 609<br>FR - A - 2 306 745<br>FR - A - 2 315 971<br>GB - A - 645 710<br>US - A - 2 349 944<br>US - A - 3 488 926</b></td><td></td></tr>
</table>

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Fermentation process and apparatus

This invention relates to a fermentation process, particularly for separating gas bubbles from an aqueous medium in which they are entrained, and also to apparatus therefor.

In continuous fermentation it is desirable to inject a gas, e.g. air or oxygen, into an aqueous medium at one or more points and to remove the gas bubbles entrained in the aqueous medium at one or more points displaced from the injection points.

Thus continuous fermentation may be conducted in an elongated vertical apparatus in which the aqeuous medium is circulated: the apparatus may have one or more chambers down which the aqueous medium is caused to flow from the top of the apparatus to the bottom thereof (such a chamber or chambers is herein referred to as the downcomer) and one or more chambers wherein the aqueous medium is caused to flow from the bottom of the apparatus to the top (such a chamber or chambers is herein referred to as the riser). The apparatus may be in the form of two coaxial tubes wherein one tube forms the riser and the other forms the downcomer. Instead of coaxial tubes a similar effect may be achieved by means of appropriately designed baffles, or by separate vessels interconnected at the top and bottom. In such apparatus the gas is injected into the downcomer and/or riser. The injection of the gas into the downcomer and/or riser may be employed to effect circulation of the aqueous medium. Such a process is described in our GB—A—1473665. On reaching the top of the riser, the aqueous medium is transferred to the top of the downcomer whence it travels down the downcomer to the bottom of the riser. If the injection of the gas is to effect circulation it is necessary that the entrained gas is removed from the aqueous medium between the top of the riser and the top of the downcomer. Also, where the gas is utilised during the passage of the aqueous medium as it circulates, it is desirable to remove the spent gas bubbles, also any bubbles of gas, e.g. carbon dioxide, produced, from the aqueous medium before it passes to the downcomer. Separation of the gas bubbles from the aqueous medium at the top of the riser may be effected by providing a pond or reservoir connecting the top of the riser to the top of the downcomer. During passage of the aqueous medium laterally through the pond from the top of the riser to the top of the downcomer, the gas bubbles rise to the surface from whence they can be removed.

It is with this type of arrangement that the present invention is concerned. In the known system, to ensure that essentially all of the gas bubbles rise to the surface during passage from the riser to downcomer, the surface area of the pond between the riser outlet and the downcomer inlet must exceed the volumetric flow rate divided by the rate of rise of the gas bubbles. An example of such a system is described in our GB—A—1353008. In that specification it is disclosed that, to assist degasification, the upper end of the partition between the riser and downcomer slopes outwards to increase the free surface area available for disengagement of the gas bubbles. It was also disclosed that this sloping surface may be provided with vents to exhaust gas bubbles accumulating on the underside of the partition to above the level of the liquid in the pond. However as the scale of operation and/or of circulation of the process is increased, it becomes increasingly difficult to provide a sufficiently large pond to effect separation even with such a sloping surface and vents to assist degasification.

In the present invention the requisite surface area of the pond is reduced by effectively providing a plurality of ponds stacked one above another. Such an arrangement fits more conveniently into a vessel such as a fermenter and is particularly advantageous where space is at a premium.

Accordingly we provide a fermentation process wherein an aqueous medium comprising a culture containing microorganisms is continuously circulated round apparatus comprising a downcomer connected at its base to a riser and bubbles of gas are injected into the aqueous medium in the downcomer and/or riser and bubbles of gas are removed from said aqueous medium between the top of the riser and the top of the downcomer characterised in that the said gas bubbles are removed by passing the aqueous medium laterally through a pond connecting the top of the riser to the top of the downcomer and having submerged therein a separator comprising a plurality of spaced plates stacked one above another so that the aqueous medium passes through the spaces between the plates whereby the gas bubbles accumulate on the undersides of the plates, and venting said gas bubbles from beneath each plate to the surface of the pond.

We also provide fermentation apparatus comprising a downcomer connected at its bottom to a riser, means for injecting gas into the downcomer and/or riser, a vessel connecting the top of the riser to the top of the downcomer, the inlet to said vessel from the top of the riser being laterally displaced from the outlet to the top of the downcomer, characterised by a separator comprising a plurality of spaced plates stacked one above another so that an aqueous medium flowing in said vessel from said inlet to said outlet flows through the spaces between the plates, and means for venting gas bubbles accumulating on the undersides of each of said plates to the surface of the aqueous medium in said vessel.

In the simplest case of the present invention there are two plates stacked one above the other. Here effectively three ponds are formed; a conventional one in the space above the top plate and second and third ponds in the spaces below the upper and lower plates respectively. It is preferred to employ at least 3 plates disposed one above another thereby providing at least four ponds.

The plates may be inclined to the vertical, in the direction of flow of the aqueous medium, by an angle between 30° and 150°. Thus the plates may be horizontal or inclined upwardly or downwardly. Where the plates are horizontal, or inclined upwardly, i.e. inclined to the vertical at an angle, in the direction of flow, of between 30° and 90°, it may be desirable to provide a transverse baffle at the end of the plates on the downcomer side thereof depending downwards so as to prevent the gas bubbles accumulating at the undersides of the plates from being swept away with the flow of the aqueous medium.

The gas bubbles may be removed by means of suitable vent pipes communicating with the underside of the plates at the position where the gas bubbles accumulate.

Where the plates are inclined downwards, the gas bubbles will tend to flow back towards the riser side of the plates on the underside thereof. In such a case a vent for collection of the accumulating gas bubbles from each plate may not be necessary but may be desirable in some cases.

The plates, whether inclined or not to the horizontal in the direction of flow, are preferably inclined to the horizontal in the direction transverse to the direction of flow. In this way the gas bubbles tend to move to the higher side of the plates. This facilitates collection and removal of the accumulating gas bubbles. The angle of inclination of the plates to the transverse horizontal is preferably at least 10° and less than 60°. Even more preferably, the plates are bent or curved having two or more angles of inclination to the transverse horizontal. Thus the lower part of each plate may be inclined at a relatively low angle, e.g. between 10° and 25° while the upper part of each plate is inclined at a somewhat greater angle, e.g. between 30° and 60°. It will be appreciated that other plate configurations could be used with similar results: thus plates having an inverted — V configuration may be used — and such plates may have gas collection channels of any suitable configuration, eg inverted — U shape, at their apices. Indeed the plates may be corrugated and so have a plurality of longitudinally extending apices.

In a preferred form of the invention there are more than two plates stacked one above another. In such a case there is a tendency for proportionately more of the aqueous medium to flow through the channels provided by the upper plates than through the channels provided by the lower plates. To compensate for this, and to even out the flow rates, it is preferred that baffles are provided to restrict the outlets of the channels provided by the upper plates to a greater extent than the outlets of the channels provided by the lower plates.

Various embodiments of the present invention are illustrated by reference to the accompanying drawings wherein

Figure 1 is a side elevation of part of a first embodiment of the apparatus with part of the side cut away for clarity,

Figure 2 is a section along the line A—A of Figure 1,

Figure 3 is an end elevation of part of a second embodiment viewed from the inlet, i.e. riser, end with part of the inlet end cut away,

Figure 4 is a section along the line B—B of Figure 3,

Figure 5 is an exploded isometric view of the outlet, i.e. downcomer, end of part of a third embodiment of the apparatus,

Figures 6 and 7 are side elevations, part cut away, of parts of fourth and fifth embodiments respectively,

Figure 8 is a plan of sixth embodiment,

Figure 9 is a cross section along the line C—C of Figure 8,

Figure 10 is an isometric view of one of the segments from which the plates used in the embodiment of Figures 8 and 9 are constructed.

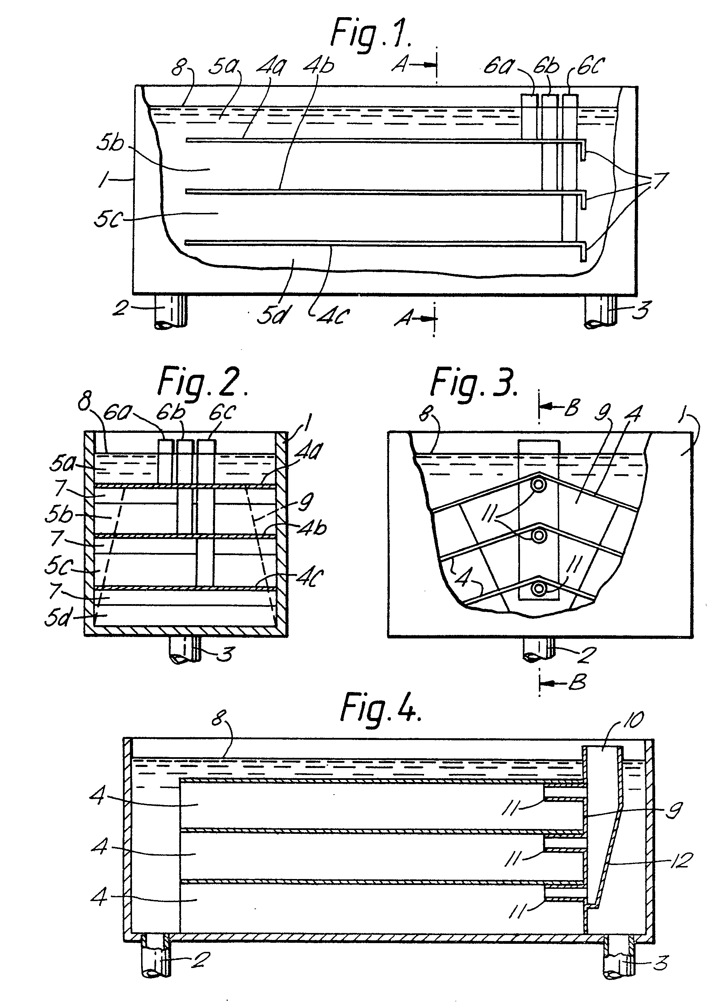

In the apparatus of the embodiment shown in Figures 1 and 2, there is provided, between the top of the riser and the top of the downcomer, a vessel 1 provided with an inlet 2 from the riser and an outlet 3 to the downcomer. A number of horizontal plates 4a, 4b, 4c, (for simplicity only three plates are shown in Figures 1 and 2 although it will be appreciated that larger numbers may be employed) are disposed in the vessel between the inlet 2 and the outlet 3, thereby forming channels 5a, 5b, 5c, 5d for the passage of the aqueous medium. The plates 4a, 4b, 4c are provided at the outlet end with vent pipes 6a, 6b, 6c respectively and with baffles 7.

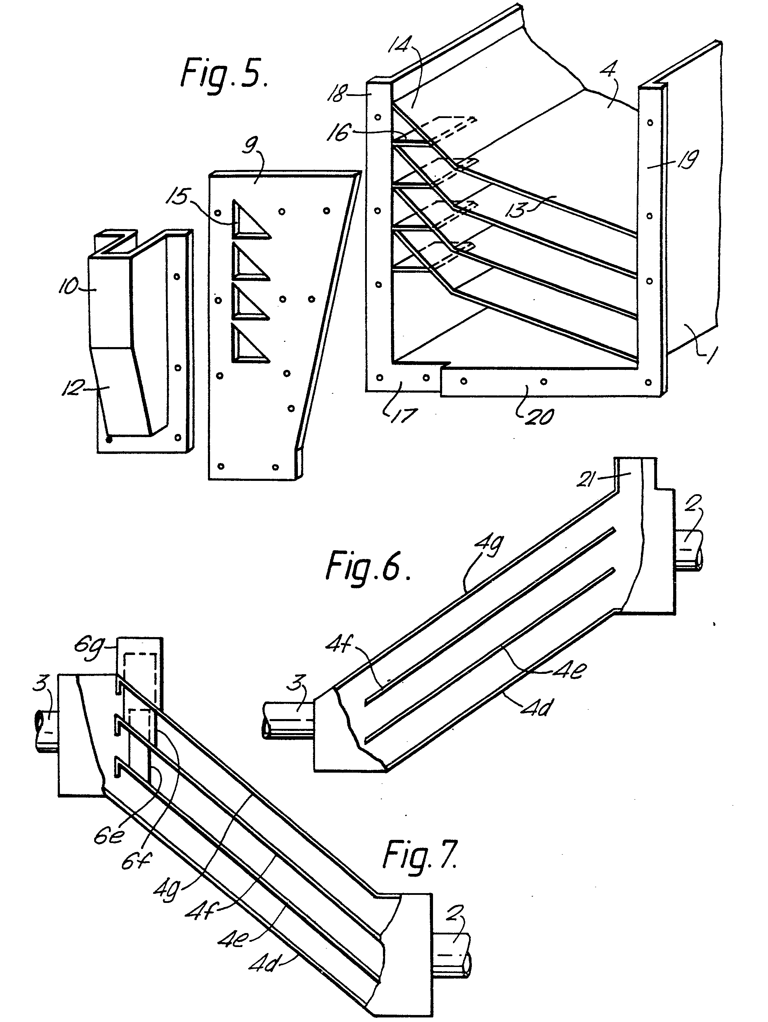

The gas/liquid mixture is fed to the separator through inlet 2 and the degassed aqueous medium is removed via outlet 3. The level 8 of the aqueous medium is maintained above the uppermost plate 4a so that all the plates are submerged in the mixture. On passage of the aqueous medium from the inlet 2 to the outlet 3, gas entrained in the aqueous medium rises. In the case of the aqueous medium passing through the uppermost channel 5a, the gas bubbles rise to the surface 8 as in the case of a conventional gas/liquid separator pond. However gas bubbles rising from the aqueous medium passing through the other channels 5b, 5c, 5d, i.e. beneath plates 4a, 4b, 4c, respectively, accumulate on the underside of the plates. The flow of the aqueous medium through the channels causes the bubbles to flow towards the outlet end of the plates from whence the gas is vented to above the liquid surface 8 via vent pipes 6a, 6b, 6c. (For clarity the vent pipes have been shown spaced both laterally and longitudinally in Figures 1 and 2. It will be appreciated that such spacing in two directions is not necessary: indeed as will be described hereinafter in relation to Figure 7, coaxial vent pipes could be employed). The baffles 7 serve to trap the gas bubbles accumulating beneath the plates and to stop them from being carried, by the flow of the aqueous medium, to the outlet 3.

It has been found that the volume flow rate through the channels 5a, 5b, 5c, 5d decreases as the bottom of the vessel 1 is approached. Hence as shown by dotted lines in Figure 2 (not shown in Figure 1) further baffles 9 may be provided to restrict the outlet areas of channels 5b, 5c, 5d with 5b more restricted than 5c which in turn is more restricted than 5d so that the volume flow rate through the channels is made more even. Likewise the volume flow rate in the conventional pond formed by channel 5a can be restricted by suitable baffles (not shown).

In the embodiment illustrated in Figures 3 and 4, the plates 4 have an inverted — V configuration; each half of the plate is inclined to the horizontal transverse to the direction of flow at an angle of about 20°. In the embodiment a baffle plate 9, progressively restricting the outlet areas of the channels from the bottom of the vessel to the top, carries a vent pipe 10. This vent pipe 10 communicates with the apices of the undersides of the plates 4 via short pipes 11 extending through baffle plate 9. The reason for having the short pipes 11, as opposed to merely providing holes in the baffle plate 9, is to displace the gas collection region from baffle plate 9 and hence away from the effect of any eddy currents produced adjacent plate 9. The lower portion 12 of vent pipe 10 is tapered as less gas has to flow therethrough than through the upper part of the vent pipe 10.

In the embodiment shown in Figure 5, the plates 4 are inclined to the transverse horizontal: the lower part 13 of the plate is inclined at a relatively small angle, for example 20° while the upper part 14 is inclined at a greater angle, for example 45°. The vent pipe 10 is attached to a baffle plate 9 which is provided with apertures 15 corresponding to the uppermost part of the space beneath each plate 4. A small horizontal plate 16 is provided at the outlet end of the upper part of the underside of plates 4 to displace the vent orifice from the effect of eddy currents adjacent to plate 9. As in the embodiment of Figures 3 and 4, the lower portion 12 of the vent pipe 10 is tapered.

The outlet end of the vessel 1 is provided with flanges 17, 18 to which the baffle plate 9 and the vent pipe 10 may be fastened, and further flanges 19, 20 to which the outlet connection (not shown) for the degassed aqueous medium may be attached.

In operation of the embodiments of Figures 3, 4 and 5 as the bubbles of gas rise and encounter the undersides of the inclined plates 4, they are directed sideways to the upper part of the inclined plates from whence they are removed via the vent pipe 10 at the outlet end of the plates.

In the embodiment shown in Figure 6, the plates 4d, 4e, 4f, 4g are inclined downwardly in the direction of flow of the aqueous medium from the inlet 2 to the outlet 3. Thus the plates are inclined to the vertical in the direction of flow by an obtuse angle, conveniently about 125°. In this embodiment the bottom plate 4d merely forms the base of vessel 1 while the top plate 4g forms the top of the vessel. As the top plate 4g is not submerged in the aqueous medium, this plate merely corresponds to a lid on a conventional pond. Furthermore as there is no provision for flow of aqueous medium beneath plate 4d, neither plate 4d nor plate 4g are submerged and hence are discounted when computing the number of submerged plates as required by the invention. Thus in this embodiment as shown in Figure 6, there are two submerged plates, viz the intermediate plates 4e and 4f.

As the aqueous medium flows from the inlet 2 to the outlet 3, the gas bubbles rise and, by gravity, tend to travel back towards the inlet 2 along the inclined underside of the plates. On reaching the inlet end of the plates, the accumulated gas bubbles rise and are vented from the system via a vent pipe 21. In the embodiment shown in Figure 7, the plates are inclined upwardly in the direction of flow. Thus they are inclined to the vertical, in the direction of flow, at an acute angle, preferably at least 30°C. (It will be appreciated that when the angle in inclination to the vertical is 90°, the plates are horizontal and hence the system is similar to the embodiment of Figures 1 and 2).

Again only the intermediate plates 4e, 4f are submerged plates in accordance with the invention. Baffles 7 are provided, as in the embodiment of Figures 1 and 2, to stop accumulating gas from being swept to the outlet 3 by the flow of the aqueous medium. In this embodiment, the vent means comprises a coaxial series of pipes 6e, 6f, 6g extending from the undersides of plates 4e, 4f 4g. While the vent pipes may all extend to above the top of plate 4g, it is only necessary that the vent pipe 6e extends to above plate 4f and that vent pipe 6f extends to above plate 4g.

In both the embodiments of Figures 6 and 7, venting is facilitated if, in addition to being inclined to the vertical in the direction of flow, the plates are also inclined to the transverse horizontal, for example as in the embodiments previously described in relation to Figures 3 to 5.

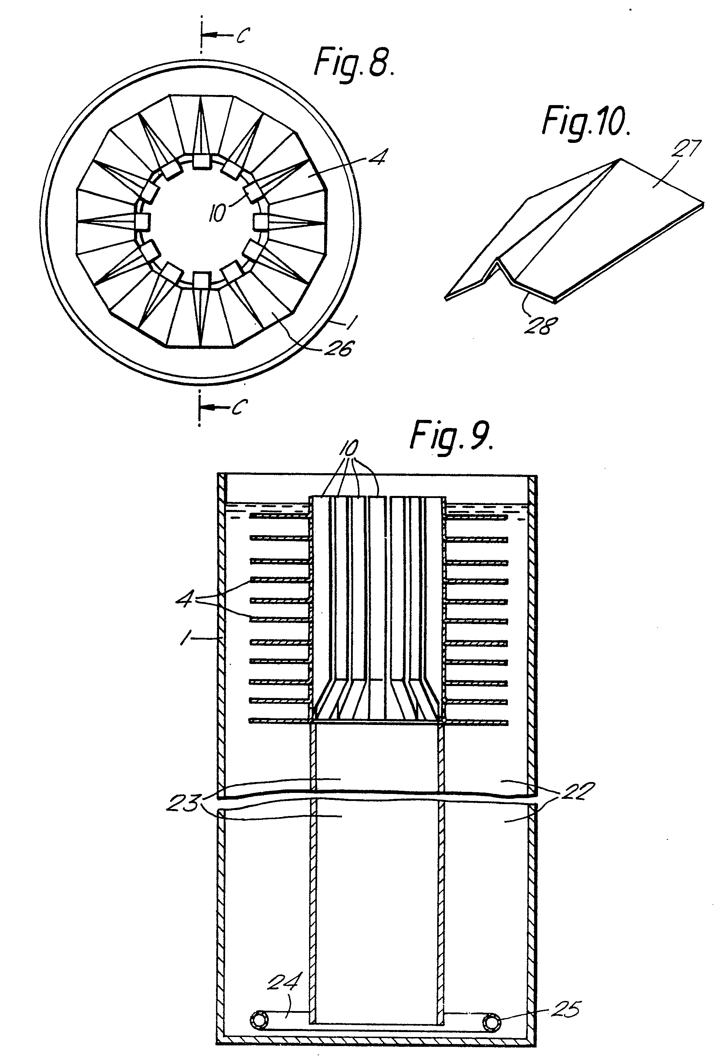

Figures 8 to 10 illustrate the application of the invention to a cylindrical fermenter provided with a riser 22 coaxially disposed round a downcomer 23. The bottom of the downcomer 23 stops short of the bottom of the fermenter so that the aqueous medium can pass from downcomer 23 into riser 22 through a gap 24. A sparge ring 25 is provided at the bottom of the riser 22 to inject air into the aqueous medium therein. Feed pipes (not shown) are provided to introduce the fermentation substrate and fresh aqueous medium while a further pipe (not shown) is provided to continuously draw off the product.

The plates 4 are of annular configuration and are each built up from twelve individual segments 26. Alternatively the plates may be constructed as integral sheets. The segments have a corrugated configuration of the type shown in Figure 10. At the inlet, i.e. riser, end 27 of the segments, the configuration is an inverted — V shape, e.g of the type shown in Figure 3, while at the inner, i.e. downcomer, end 28, the segments have an inverted double — V configuration akin in cross-section to that of plates of the type shown in the embodiment of Figure 5 joined side by side. It will be appreciated that, instead of the inverted double — V shaped at the inner end 28, the segments may be constructed such that the "V" is sharper, i.e the angle of inclination to the transverse horizontal may increase, as the inner end 28 is approached. For clarity, the corrugations are not shown in Figure 9. A series of vent pipes 10 are situated at the inner edge of the annular plates 4.

The invention is of particular utility in fermenters for producing single cell protein by fermentation of a suitable substrate, e.g. methanol, using an appropriate micro-organism, preferably a bacterium.

**Claims**

1. A fermentation process wherein an aqueous medium comprising a culture containing microorganisms is continuously circulated round apparatus comprising a downcomer connected at its base to a riser and bubbles of gas are injected into the aqueous medium in the downcomer and/or riser and bubbles of gas are removed from said aqueous medium between the top of the riser and the top of the downcomer characterised in that said gas bubbles are removed by passing the aqueous medium laterally through a pond connecting the top of the riser to the top of the downcomer and having submerged therein a separator comprising a plurality of spaced plates stacked one above another so that the aqueous medium passes through the spaces between the plates whereby the gas bubbles accumulate on the undersides of the plates, and venting said gas bubbles from beneath each plate to the surface of the pond.

2. A fermentation apparatus for liquid circulation and gas separation which comprises a downcomer connected at its bottom to a riser, means for injecting gas into the downcomer and/or riser, a vessel (1) connecting the top of the riser to the top of the downcomer, the inlet (2) to said vessel from the top of the riser being laterally displaced from the outlet (3) to the top of the downcomer, characterised by a separator comprising a plurality of spaced plates (4) stacked one above another so that an aqueous medium flowing in said vessel from said inlet to said outlet flows through the spaces between the plates, and means (6; 109, 11; 21) for venting gas bubbles accumulating on the undersides of each of said plates to the surface of the aqueous medium in said vessel.

3. An apparatus according to claim 2 wherein the separator comprises at least three plates (4).

4. An apparatus according to claim 2 or claim 3 wherein the plates (4) are inclined to the vertical, in the direction of flow of the aqueous medium, by an angle between 30° and 150°.

5. An apparatus according to any one of claims 2 to 4 wherein the plates (4) are inclined to the horizontal in the direction transverse to the direction of flow of the aqueous medium by an angle between 10° and 60°.

6. An apparatus according to claim 5 wherein the plates (4) are bent or curved to have two or more angles of inclination to the transverse horizontal.

7. An apparatus according to claim 2 or claim 3 wherein the plates (4) are of inverted — V configuration.

**Patentansprüche**

1. Gärverfahren, bei dem ein wäßriges Medium, das eine Mikroorganismen enthaltende Kultur enthält, kontinuierlich in einer Vorrichtung, die ein Fallrohr aufweist, das an seinem Unterteil bzw. Boden mit einem Steigrohr verbunden ist, im Kreislauf herumgeführt wird, bei dem Gasblasen in das in dem Fallrohr und/oder dem Steigrohr befindliche, wäßrige Medium eingeblasen werden und bei dem aus dem wäßrigen Medium zwischen dem Oberteil des Steigrohrs und dem Oberteil des Fallrohrs Gasblasen entfernt werden, dadurch gekennzeichnet, daß die Gasblasen entfernt werden, indem das wäßrige Medium seitlich durch das in das Oberteil des Steigrohrs mit dem Oberteil des Fallrohrs verbindendes Becken hindurchgeleitet wird, wobei in das Becken ein Abscheider, der eine Vielzahl von in Abständen voneinander angeordneten und übereinander gestapelten bzw. gelagerten Platten aufweist, eingetaucht ist, so daß das wäßrige Medium durch die Zwischenräume zwischen den Platten hindurchströmt, wodurch sich die Gasblasen an der Unterseite der Platten ansammeln, und daß die Gasblasen von der Unterseite jeder Platte zu der Oberfläche des Beckens abgelassen werden.

2. Gärvorrichtung zur Flüssigkeitsumwälzung und Gasabtrennung mit einem an seinem Unterteil bzw. Boden mit einem Steigrohr verbundenen Fallrohr, Einrichtungen zum Einblasen von Gas in das Fallrohr und/oder das Steigrohr und einem das Oberteil des Steigrohrs mit dem Oberteil des Fallrohrs verbindenden Behälter (1), wobei der Einlaß (2) vom Oberteil des Steigrohrs zu dem Behälter gegenüber dem Auslaß (3) zum Oberteil des Fallrohrs seitlich verschoben ist, gekennzeichnet durch einen Abscheider, der eine Vielzahl von in Abständen voneinander angeordneten und übereinander gestapelten bzw. gelagerten Platten (4) aufweist, so daß ein wäßriges Medium, das in dem Behälter von dem Einlaß zu dem Auslaß strömt, durch die Zwischenräume zwischen den Platten hindurchströmt, und Einrichtungen (6; 10, 11; 21) zum Ablassen von Gasblasen, die sich an den Unterseiten aller Platten ansammeln, zu der Oberfläche des in dem Behälter befindlichen, wäßrigen Mediums.

3. Vorrichtung nach Anspruch 2, bei der der Abscheider mindestens drei Platten (4) aufweist.

4. Vorrichtung nach Anspruch 2 oder Anspruch 3, bei der die Platten (4) in der Strömungsrichtung des wäßrigen Mediums gegenüber der Senkrechten um einen Winkel zwischen 30° und 150° geneigt sind.

5. Vorrichtung nach einem der Ansprüche 2 bis 4, bei der die Platten (4) in Querrichtung zu der Strömungsrichtung des wäßrigen Mediums gegenüber der Waagerechten um einen Winkel zwischen 10° und 60° geneigt sind.

6. Vorrichtung nach Anspruch 5, bei der die Platten (4) gebogen oder gekrümmt sind, so daß sie gegenüber der Waagerechten in Querrichtung zwei oder mehr Neigungswinkel aufweisen.

7. Vorrichtung nach Anspruch 2 oder Anspruch 3, bei der die Platten (4) die Gestalt eines umgekehrten V haben.

## Revendications

1. Procédé de fermentation selon lequel un milieu aqueux constitué par une culture contenant des micro-organismes subit une circulation en continu à l'intérieur d'un appareil comprenant une collone descendante reliée à sa base à une colonne montante, et selon lequel des bulles de gaz sont injectées dans le milieu aqueux dans la colonne descendante et/ou dans la colonne montante, des bulles de gaz étant éliminées de ce milieu aqueux entre le sommet de la colonne montante et le sommet de la colonne descendante, caractérisé en ce que ces bulles de gaz sont éliminées par passage du milieu aqueux latéralement à travers un réservoir reliant le sommet de la colonne montante au sommet de la colonne descendante et dans lequel est immergé un séparateur comprenant plusieurs plaques espacées empilées l'une au-dessus de l'autre, de sorte que le milieu aqueux passe par les espaces ménagés entre les plaques et que les bulles de gaz s'accumulent sur les faces inférieures des plaques, et en ce qu'on évacue ces bulles de gaz depuis le dessous de chaque plaque en direction de la surface du réservoir.

2. Appareil de fermentation pour la circulation d'un liquide et la séparation d'un gaz comprenant une colonne descendante reliée à sa base à une colonne montante, des moyens pour injecter un gaz dans la colonne descendante et/ou la colonne montante, un récipient (1) reliant le sommet de la colonne montante au sommet de la colonne descendante, l'entrée (2) dans ce récipient depuis le sommet de la colonne montante étant écartée latéralement de la sortie (3) reliée au sommet de la colonne descendante, caractérisé par un séparateur comprenant plusieurs plaques espacées (4) empilées l'une au-dessus de l'autre, de sorte qu'un milieu aqueux s'écoulant dans ce récipient de l'entrée vers la sortie passe à travers les espaces ménagés entre les plaques, et des moyens (6; 10, 11; 21) pour évacuer les bulles de gaz s'accumulant sur les faces inférieures de chacune de ces plaques en direction de la surface du milieu aqueux qui se trouve dans ce récipient.

3. Appareil suivant la revendication 2, caractérisé en ce que le séparateur comprend au moins trois plaques (4).

4. Appareil suivant la revendication 2 ou 3, caractérisé en ce que les plaques (4) sont inclinées sur la verticale dans la direction d'écoulement du milieu aqueux selon un angle compris entre 30° et 150°.

5. Appareil suivant l'une quelconque des revendications 2 à 4, caractérisé en ce que les plaques (4) sont inclinées sur l'horizontale dans la direction transversale à la direction d'écoulement du milieu aqueux selon un angle compris entre 10° et 60°.

6. Appareil suivant la revendication 5, caractérisé en ce que les plaques (4) sont repliées ou incurvées de façon à présenter deux ou plusieurs angles d'inclinaison par rapport à l'horizontale dans le sens transversal.

7. Appareil suivant la revendication 2 ou 3, caractérisé en ce que les plaques (4) ont une configuration en V inversé.

# Fig.1.

# Fig.2.

# Fig.3.

# Fig.4.

1

Fig.5.

Fig.6.

Fig.7.

0 015 680

## Fig. 8.

## Fig.10.

## Fig. 9.

3